**Europäisches Patentamt**

**European Patent Office**

**Office européen des brevets**

(19)

(11) **EP 0 868 204 B1**

(12) **EUROPÄISCHE PATENTSCHRIFT**

(45) Veröffentlichungstag und Bekanntmachung des Hinweises auf die Patenterteilung:
**09.04.2003 Patentblatt 2003/15**

(51) Int Cl.$^7$: **A61M 13/00**

(21) Anmeldenummer: **96942262.5**

(22) Anmeldetag: **22.11.1996**

(86) Internationale Anmeldenummer:
**PCT/DE96/02252**

(87) Internationale Veröffentlichungsnummer:
**WO 97/018848 (29.05.1997 Gazette 1997/23)**

(54) **VORRICHTUNG ZUM INSUFFLIEREN VON GAS IN EINE KÖRPERHÖHLE EINES MENSCHLICHEN ODER TIERISCHEN KÖRPERS**

DEVICE FOR INSUFFLATING GAS INTO A CAVITY IN A HUMAN OR ANIMAL BODY

DISPOSITIF POUR INSUFFLER DU GAZ DANS UNE CAVITE DU CORPS D'UN ETRE HUMAIN OU ANIMAL

(84) Benannte Vertragsstaaten:
**DE FR GB IT**

(30) Priorität: **22.11.1995 DE 19543561**

(43) Veröffentlichungstag der Anmeldung:
**07.10.1998 Patentblatt 1998/41**

(73) Patentinhaber: **Storz Endoskop GmbH**
**8200 Schaffhausen (CH)**

(72) Erfinder:
• **GORD, John, C.**
  **Venice, CA 90291 (US)**

• **JONES, Eric, M.**
  **Southbridge, MA 01550 (US)**
• **KRAFT-KIVIKOSKI, Jürgen**
  **D-78315 Radolfzell (DE)**

(74) Vertreter: **Münich, Wilhelm, Dr.**
**Dr. Münich & Kollegen**
**Anwaltskanzlei**
**Wilhelm-Mayr-Strasse 11**
**80689 München (DE)**

(56) Entgegenhaltungen:
**EP-A- 0 489 716       WO-A-95/23006**

## Beschreibung

### Technisches Gebiet

Die Erfindung bezieht sich auf eine Vorrichtung zum Insufflieren von Gas, wie beispielsweise $CO_2$ in eine Körperhöhle eines menschlichen oder tierischen Körpers gemäß dem Oberbegriff des Patentanspruchs 1.

Derartige Vorrichtungen werden u.a. bei endoskopischen Operationen benötigt.

### Stand der Technik

Gattungsgemäße Vorrichtungen zum Insufflieren von Gas sind in einer Vielzahl von Ausführungsformen bekannt. Hierzu wird beispielsweise auf die DE-A-30 00 218, die EP-B-0 169 972 oder die DE-C-36 11 018 bzw. die US-PS 4 874 362 oder die DE-A-42 19 859 verwiesen. Auf diese Druckschriften wird im übrigen zur Erläuterung aller hier nicht näher beschriebenen Einzelheiten ausdrücklich Bezug genommen.

Alle bekannten gattungsgemäßen Vorrichtungen weisen einen Anschluß für eine Gasquelle, beispielsweise eine Druckflasche, und die Möglichkeit zum Einstellen des Gasdruckes bzw. des -flusses auf. Hierzu können die bekannten Vorrichtungen beispielsweise einen Druckregler, der den Druck der Gasquelle auf einen typischerweise zwischen 0 und 50 mm Hg einstellbaren Insufflationsdruck (Soll-Gasdruck) mindert, und einen Flußregler aufweisen, der den Gasfluß auf einen einstellbaren Wert (Soll-Gasfluß) einstellt.

Bei den in den vorstehend genannten Druckschriften beschriebenen gattungsgemäßen Vorrichtungen kann der Gasdruck bzw. der Gasfluß nicht nur gesteuert, sondern auch geregelt werden. Hierzu verfügen diese Vorrichtungen über eine Meßeinrichtung, die Sensoren für den Ist-Gasdruck und den Ist-Gasfluß aufweist, und eine Steuereinheit, an der die Ausgangssignale dieser Sensoren anliegen, und die den Druck- und den Flußregler steuert.

Problematisch bei den bekannten Vorrichtungen zum Insufflieren von Gas in eine Körperhöhle ist jedoch die Messung des Gasdruckes, da es in der Regel nicht oder nur mit großem Aufwand möglich ist, den Gasdruck direkt in der Körperhöhle zu messen. Durch die Messung des Druckes außerhalb der Körperhöhle entsteht aufgrund des strömenden Gases und des (u.a. hierdurch hervorgerufen) Druckabfalles ein Meßfehler, der bei medizinischen Anwendungen nicht ohne weiteres toleriert werden kann.

Es ist deshalb vorgeschlagen worden, zur Messung des "statischen" Gasdruckes p den Gasfluß Q auf den Wert 0 "herunterzufahren" und den Gasdruck dann zu messen, wenn der Gasfluß tatsächlich "statisch" den Wert 0 erreicht hat. Hierzu wird beispielsweise auf die Fig. 6 der US-PS 4 874 362 und die zugehörige Beschreibung oder auf den Anspruch 1 der DE-A-30 00 218 und die zugehörige Beschreibung verwiesen. Diese Vorgehensweise hat zwar den Vorteil, daß sie relativ genau die Messung des Gasdruckes erlaubt, nachteilig ist jedoch, daß sie keine auch nur wenigstens einigermaßen konstante Gaszufuhr erlaubt.

Weiterhin ist vorgeschlagen worden, unter Berücksichtigung des Strömungswiderstands des Schlauchbestecks und des Insufflationsinstruments, wie beispielsweise einer Verres-Nadel, die sich zwischen dem Drucksensor und der Körperhöhle, deren Druck bestimmt werden soll, aus dem Ausgangssignal des Sensors den tatsächlich in der Körperhöhle herrschenden Druck zu berechnen.

In der WO 95/23006 wird eine Insufflationsvorrichtung vorgeschlagen, welche unmittelbar nach einer Ruhestellung bei konstantem Gasdurchfluß den Druckabfall $\Delta p$ ermittelt. Dieser hier ermittelte Druckabfall $\Delta p$ wird für die weitere Insufflationsphase zur Abschätzung des Drucks in der Körperhöhle verwendet da die Abschätzung des Druckabfalls $\Delta p$ auf einer Messung mit konstantem Gasfluß Q basiert. Bei Änderungen des Gasflußes bei den nachfolgenden Insufflationsphasen wird der entsprechend geänderte Druckabfall neu berechnet.

In der Praxis bereitet jedoch die Bestimmung des Strömungswiderstandes und damit des Druckabfalls zwischen dem Drucksensor und der Körperhöhle Schwierigkeiten.

### Darstellung der Erfindung.

Der Erfindung liegt die Aufgabe zugrunde, eine Vorrichtung zum Insufflieren von Gas in eine Körperhöhle eines menschlichen oder tierischen Körpers gemäß dem Oberbegriff des Patentanspruchs 1 derart weiterzubilden, daß der Druckabfall zwischen Drucksensor und Körperhöhle und damit der Druck in der Körperhöhle so genau wie möglich bei höchstmöglicher Patientensicherheit bestimmt werden kann.

Eine erfindungsgemäße Lösung dieser Aufgabe ist im Patentanspruch 1 angegeben. Weiterbildungen der Erfindung sind Gegenstand der Ansprüche 2 folgende.

Die Erfindung geht von einer gattungsgemäßen Verrichtung zum Insufflieren von Gas in eine Körperhöhle eines menschlichen oder tierischen Körpers aus und bildet diese Vorrichtung erfindungsgemäß wie folgt weiter:

Die Steuereinheit verwendet zur Berechnung des Druckabfalls Pdrop zwischen dem Ort des Drucksensors und der Körperhöhle, deren Druck als tatsächlicher Gasdruck Pabd (intraabdominaler Druck) der relevante Druck ist, aus dem außerhalb der Körperhöhle gemessenen Gasdruck Pfill und dem gemessenen Gasfluß Q stückweise Funktionen, aufgrund derer der berechnete Druckabfall pdrop als Funktion von Q kleiner oder höchstens gleich dem tatsächlichen Druckabfall ist. Weiterhin berechnet die Steuereinheit Ereignisund/oder Zeit-gesteuert während der normalen Insufflation laufend den Gasdruck Pabd und regelt aufgrund des berechneten Gasdruckes Pabd die Insufflation.

Aufgrund der erfindungsgemäßen Vorgehensweise wird der in der Körperhöhle herrschende intraabdominale Druck zu keinem Zeitpunkt unterschätzt. Da laufend der in der Körperhöhle herrschende intraabdominale Druck bestimmt wird, kann es - im Gegensatz zu bekannten Vorrichtungen, bei denen nur in vergleichsweise großen Zeitabständen eine Bestimmung des intraabdominalen Drucks erfolgt - niemals zu einem zu hohen Druck in der Körperhöhle kommen.

Untersuchungen haben ergeben, daß es bei üblichen Gasflüssen von bis zu 20 Liter/min., wie sie bei medizinischen Insufflationsvorgängen verwendet werden, ausreichend ist, zwei Funktionen für die Bestimmung des Druckabfalls zu verwenden, die jeweils für bestimmte Flußraten herangezogen werden:

So ist es gemäß Anspruch 2 bevorzugt, wenn bei Flußraten, die kleiner als eine bestimmte Flußrate Qums sind, eine quadratische Abhängigkeit des Druckabfalls von der Flußrate verwendet wird. Bei Flußraten, die größer als diese bestimmte Flußrate sind, ist es bevorzugt, wenn eine lineare Abhängigkeit des Druckabfalls von der Flußrate verwendet wird. Durch die Verwendung dieser beiden Funktionen ist gewährleistet, daß der berechnete Druckabfall immer kleiner oder höchstens gleich dem tatsächlich auftretenden Druckabfall zwischen Meßstelle und Körperinneren ist, so daß zu keinem Zeitpunkt in der Körperhöhle ein zu hoher Druck auftreten kann.

Das erfindungsgemäße Prinzip zur Bestimmung des Druckabfalls in der Schlauchleitung und dem sonstigen Instrumentarium in Abhängigkeit von der Flußrate kann bei den verschiedensten Vorrichtungen angewandt werden:

So ist es beispielsweise bei Vorrichtungen anwendbar, bei denen die Steuereinheit die konstanten K1 und K2 unter Berücksichtigung des Strömungswiderstandes des Insufflationsbesteckes bestimmt. Insbesondere ist es denkbar, daß diese Konstanten aufgrund von Erfahrungswerten und der jeweiligen Länge des Schlauchbestecks sowie des verwendeten Instruments berechnet werden (Anspruch 3).

Besonders vorteilhaft ist es jedoch, wenn gemäß Anspruch 4 die Steuereinheit zur Bestimmung der Konstanten K1 und K2 die (in die Gleichung gemäß Anspruch 1 bei Auflösung dieser Gleichungen nach den Konstanten K1 und K2 einzusetzenden) Werte Pfill und Q bei im wesentlichen konstanten Gasfluß Q und den Wert Pabd bei einem Gasfluß ermittelt, der gegenüber dem normalen Gasfluß "deutlich abgesenkt" ist.

Der Fluß kann dabei auf "Null" oder auf einen deutlich von Null verschiedenen Wert abgesenkt werden, wie dies in der PCT Anmeldung WO-A-9 534 336 beschrieben ist (Anspruch 4).

In dieser Anmeldung ist beschrieben, daß der Ist-Gasfluß während der Druckmessung auf einen Wert zwischen 10 und 40 % des Soll-Gasflusses eingestellt wird (Anspruch 5).

In beiden Fällen wird für die Korrektur des von dem Drucksensor gemessenen Druckwertes der Gasfluß vor der Absenkung, also der Gasfluß während des "normalen" Insufflationsbetriebes herangezogen.

Da sich der Strömungswiderstand des Insufflationsbesteckes - beispielsweise aufgrund eines unbeabsichtigten Knicks in der Leitung - ändern kann, ist es gemäß Anspruch 6 bevorzugt, wenn die Steuereinheit die Konstanten K1 und K2 für die Berechnung des intraabdominalen Drucks in der Körperhöhle lediglich für den nächsten Zyklus, d. h. bis zur nächsten Flußabsenkung benutzt. Die Steuereinheit berechnet deshalb bei diesem bevorzugten Ausführungsbeispiel die Konstanten K1 und K2 für jeden Zyklus aus den beim vorherigen Zyklus ermittelten Werten neu.

Im Anspruch 7 ist eine Möglichkeit angegeben, wie der Wert des Flusses bzw. der Flußrate Qums ermittelt wird, bei der die Steuereinheit zwischen linearer und quadratischer Abhängigkeit des Drucksabfalles von der Flußrate umschaltet. Dabei wird der Wert der Flußrate, bei dem umgeschaltet wird, durch den Schnittpunkt der linearen und der quadratischen Abhängigkeit des Druckabfalls Pdrop von der Flußrate Q für die jeweils ermittelten Konstanten K1 und K2 bestimmt.

Das Meßprinzip der erfindungsgemäßen Vorrichtung ist bei den verschiedensten Vorrichtungen zum Insufflieren von Gas in Körperhöhlen anwendbar. Besonders vorteilhaft ist das Meßprinzip bei Vorrichtungen, die auch hohe Flußraten ( > 10 1/min.) erzeugen können, da bei derartigen Vorrichtungen der Einfluß der Flußrate aufgrund der großen Variationsbreite der Flußrate besonders groß ist:

Geht man z.B. von einer maximalen Flußrate von 30 1/min. und dem beim Stand der Technik üblichen Intervall von ca. 2 Sekunden zwischen zwei aufeinanderfolgenden Messungen des Körperinnendrucks bei einem auf 0 abgesenkten Gasfluß aus, so kann besonders bei einer kleinen Körperkavität bzw. Körperhöhle (kleinwüchsige Patienten, Kinder), die in diesem Falle typischerweise kleiner als 3 Liter ist, ein erheblicher Druckanstieg erfolgen, da innerhalb von 2 Sekunden ein Gasvolumen von einem Liter in die Körperkavität gefördert wird.

Die erfindungsgemäße Vorrichtung berechnet mittels der angenäherten stückweise verwendeten Druckabfall-Kennlinien zwischen den jeweiligen Druckmeßpunkten bei einem geringen Fluß oder dem Fluß 0 laufend den Druck im Körperinneren hoch, so daß ein ereignisgesteuerter vorzeitiger Abbruck der Insufflationsphase möglich ist. Es ist damit nicht notwendig, wie beim Stand der Technik, bei dem eine Zeitsteuerung der Druckmessung erfolgt, den Fluß nach einem festen Zeitintervall abzusenken, um den intraabdominalen Druck messen zu können:

Damit erhält man einen über längere Zeiträume konstanten oder wenigstens annähernd konstanten Gasfluß. Durch die gegenüber dem Stand der Technik verringerten Schwankungen des Gasflusses ändern sich auch die Ableitbedingungen weniger als beim Stand der Technik. Dennoch ist eine genaue Druckbestimmung möglich.

In diesem Zusammenhang ist es auch von Vorteil, wenn gemäß Anspruch 8 die Steuereinheit einen Alarm erzeugt, wenn der Flußsensor den Wert 0 des Gasflusses mißt, da dieser Wert auf einen Defekt des Instrumentariums hindeuten kann.

Gemäß Anspruch 9 kann weiterhin in an sich bekannter Weise zur Begrenzung des intraabdominalen Drucks ein Entlüftungsventil vorgesehen sein, das sich insbesondere am proximalen Teil eines in die Körperhöhle eingesetzten Instruments befinden kann (Anspruch 10).

In diesem Falle ist es ferner bevorzugt, wenn die Steuereinheit zur Überprüfung des Körperdrucks das Entlüftungsventil in bestimmten Zeitabständen schließt (Anspruch 11), wobei diese Zeitabstände insbesondere in Abhängigkeit vom intraabdominalen Druck gewählt werden können, also nicht fest vorgegeben sein müssen.

Weiterhin ist es - in ebenfalls bekannter Weise - bevorzugt, wenn eine Absaugpumpe zur Erhöhung der Absaugleistung vorgesehen ist (Anspruch 12).

Aufgrund der erfindungsgemäßen Ausbildung, bei der die Steuereinheit laufend den Druck im Körperinneren hochrechnet, ist es möglich, die Leistung der Absaugpumpe in Abhängigkeit vom intraabdominalen Druck zu regeln (Anspruch 13).

Weiterhin ist es aufgrund der vorgenannten erfindungsgemäßen Ausbildung möglich, als Entlüftungsventil nicht nur ein "Ein/Aus-Ventil", sondern ein Proportionalventil zu verwenden, dessen Stellung die Steuereinheit in Abhängigkeit vom Druck im Körperinneren regelt.

Eine weitere vorteilhafte Anwendung des erfindungsgemäßen Meßprinzips ist bei Vorrichtungen gegeben, bei denen in an sich bekannter Weise eine Heizeinrichtung vorgesehen ist, die das zu insufflierende Gas auf eine vorgebbare Temperatur erwärmt (Anspruch 15). Diese Heizeinrichtung kann beispielsweise in der Vorrichtung selbst, in bzw. an der Schlauchleitung zwischen der eigentlichen Vorrichtung, d.h. zwischen dem Steuergerät und dem in den Körper eingesetzten Instrument, und/oder in bzw. an dem in den Körper eingesetzten Instrument bzw. Besteck vorgesehen sein. Zur letztgenannten Ausführungsform, die besonders vorteilhaft ist, wird auf die WO 94/ 28952 verwiesen.

In Falle einer Vorrichtung, bei der das insufflierte Gas beheizt wird, ist es von besonderem Vorteil, wenn die Steuereinheit den Gasdruck und den Gasfluß unter Berücksichtigung der vorgegebenen Temperatur des Gases einstellt (Anspruch 16). Hierdurch wird die Zeitspanne bis zum Erreichen der Soll-Werte reduziert; darüberhinaus können die Meßwerte entsprechend der eingestellten Temperatur korrigiert werden, so daß die Soll/Ist-Wert-Führung verbessert wird.

Das erfindungsgemäße Meßprinzip erlaubt zusätzlich noch eine weitere Messung, nämlich die Bestimmung des Strömungs-Leitwertes der Leitungen, d.h. insbesondere der Insufflationsleitung und des Insufflationskanals des endoskopischen Instrumentariums:

Hierzu ermittelt die Steuereinheit aus den bei wenigstens zwei verschiedenen Flußwerten vom Drucksensor gemessenen Gasdrücken den Leitwert der Leitungen bis zur Körperhöhle. Mit dem Leitwert ist es ebenfalls möglich, aus dem außerhalb des Körpers gemessen Wert des Gasdruckes den Istwert des Druckes in der Körperhöhle zu berechnen.

Diese Vorgehensweise hat den Vorteil, daß eine exakte Bestimmung des - flußabhängigen - Leitwertes ohne Entnahme des Instruments aus dem Körper und damit eine unabhängige Berechnung des Druckes in der Körperhöhle das Instrument zur zusätzlichen Kontrolle der erfindungsgemäß ermittelten Werte möglich ist.

Für die Bestimmung des Leitwerts ist es erforderlich, wenigstens bei zwei verschiedenen Flußwerten zu messen, durch die Messung bei mehr als zwei Flußwerten kann die Genauigkeit durch Redundanzvergleiche erhöht werden.

Weiterhin ist es bei der erfindungsgemäßen Vorrichtung auch nicht erforderlich, eine zweite Leitung zur Messung des Druckes in der Körperhöhle vorzusehen (Anspruch 18).

Die erfindungsgemäße Vorrichtung kann besonders vorteilhaft mit dem im Anspruch 17 angegebenen Instrumentarium ausgestattet sein:

So kann zum Insufflieren des Gases in die Körperhöhle ein endoskopisches Besteck, wie beispielsweise eine Verres-Nadel vorgesehen sein, an der die Sensoren für Gasdruck und Gasfluß außerhalb der Körperhöhle angeordnet sind.

In jedem Falle hat die erfindungsgemäße Vorrichtung den Vorteil, daß sie gegenüber bekannten bzw. in älteren Patentanmeldungen beschriebenen Vorrichtungen bei hoher Genauigkeit der Druckmessung eine hervorragende Druckkonstanz bietet, obwohl der Gasfluß stark variieren darf.

Der sonstige Aufbau der erfindungsgemäßen Vorrichtung kann dem Aufbau bekannter Vorrichtungen zum Insufflieren von Gas in eine Körperhöhle hinsichtlich dem Anschluß für eine Gasquelle, der einer Meßeinrichtung und der beispielsweise einen Mikroprozessor aufweisenden Steuereinheit entsprechen. Insbesondere ist es möglich, den Druckregler und den Flußregler zu einem Bauteil zusammenzufassen.

## Kurze Beschreibung der Zeichnung

Die Erfindung wird nachstehend anhand eines Ausführungsbeispiels unter Bezugnahme auf die Zeichnung näher beschrieben, in der zeigen:

Figur 1    die reale Abhängigkeit des Drucksabfalls von der Flußrate, und
Figur 2    ein Druck/Zeitdiagramm für die erfindungsgemäße Vorrichtung.

## Darstellung eines Ausführungsbeispiels

Bei elektronischen Insufflatoren wird der Gasflow üblicherweise auf Null zurückgefahren, um den intraabdominalen Druck zu messen. Wird der Druck bei einem Gasflow von ungleich Null gemessen, so repräsentiert dieser Wert immer den Insufflationsdruck, der sich aus dem intraabdominalen Druck und dem Druckabfall des Zuleitungssystems zusammensetzt. Gerade bei modernen "Hi-Flow"-Insufflatoren und kleinen angeschlossenen Volumina besteht die Gefahr, daß zwischen zwei Meßzeitpunkten für den intraabdominalen Druck ein großer Überdruck entstehen kann.

Die erfindungsgemäße Vorrichtung erlaubt es, diese Überdruckzustände zu vermeiden.

Messungen mit verschiedenen Schlauchkonfigurationen haben gezeigt, daß der Druckabfall in Abhängigkeit vom Gasflow prinzipiell unterschiedliche Verläufe annehmen kann.

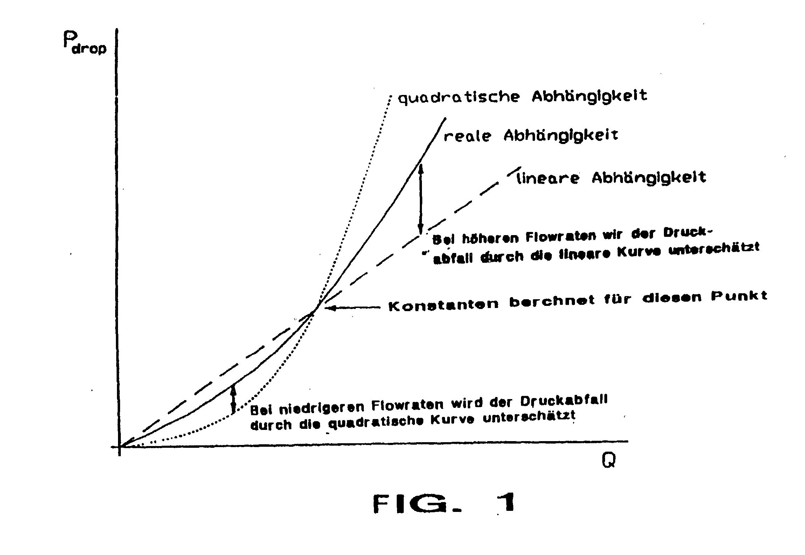

In Fig. 1 ist dies dargestellt: Die durchgezogene Kurve in Figur 1 zeigt eine typische reale Abhängigkeit des Druckabfalls $P_{drop}$ vom Gasfluß Q. Zur Annäherung dieser realen Kurve verwendet die Steuereinheit

- bei Flußraten kleiner als eine bestimmte Flußrate $Q_{ums}$ eine quadratische Abhängigkeit des Druckabfalls $P_{drop}$ von der Flußrate Q

$$P_{abd} = P_{fill} - P_{drop} = P_{fill} - K_2 * Q^2.$$

- und bei Flußraten größer als die bestimmte Flußrate $Q_{ums}$ eine lineare Abhängigkeit des Druckabfalls $P_{drop}$ von der Flußrate Q.

$$P_{abd} = P_{fill} - P_{drop} = P_{fill} - K_1 * Q$$

Die quadratische Abhängigkeit ist dabei durch die punktierte Kurve und die lineare Abhängigkeit durch die gestrichelte Kurve in Fig. 1 angegeben.

Die Faktoren $K_1$ und $K_2$ sind u.a. von der Gasart, der Temperatur des Gases, dem Durchmesser und der Länge des Instrumentariums abhängig.

Der intraabdominale Druck $P_{abd}$, der sich durch den Insufflationsdruck $P_{fill}$ minus dem Druckabfall $P_{drop}$ gegeben ist, läßt sich also auch berechnen, wenn $P_{fill}$ und der Gasflow Q gemessen werden, und das System über den Faktor k bekannt ist:

$$P_{abd} = P_{fill} - K_1 * Q \text{ für den linearen Kurvenverlauf}$$

$$P_{abd} = P_{fill} = K_2 * Q^2 \text{ für den quadratischen Kurvenverlauf.}$$

Umgekehrt lassen sich mit den gleichen Formeln die beiden Konstanten $K_1$ und $K_2$ durch Auflösung der Gleichungen nach K berechnen. Pabd und Pfill können allerdings nicht zum gleichen Zeitpunkt gemessen werden.

Vom Steuerprogramm der Steuereinheit werden deshalb die beiden Konstanten nach jedem Füllzyklus neu berechnet. Hierzu werden für Pfill und Q Werte eingesetzt, die während des abgelaufenen Zyklus abgespeichert wurden.

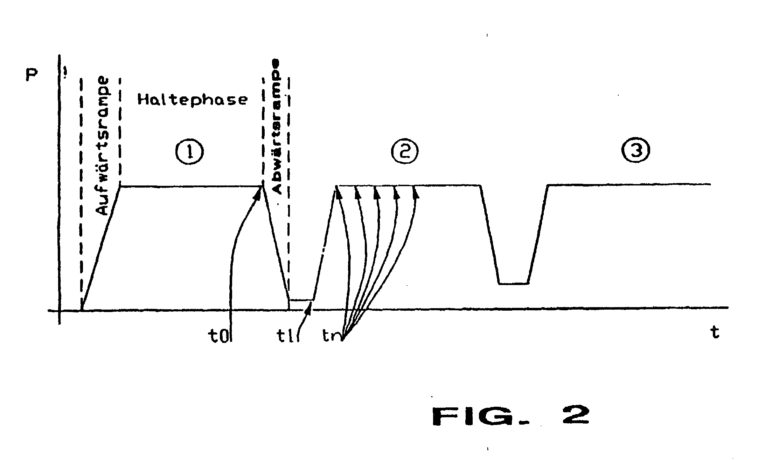

Figur 2 zeigt den zeitlichen Verlauf für die Druckvorausberechnung in einem Druck/Zeit-Diagramm.

Zum Zeitpunkt t0 werden die, für die Konstantenberechnung notwendigen Größen Pfill und Q abgespeichert. Zum Zeitpunkt t1 ist der gemessene Druckwert gleich Pabd, da der Gasflow Q=0 ist. Zu diesem Zeitpunkt liegen also alle erforderlichen Parameter vor, so daß die beiden Konstanten K1 und K2 berechnet werden können.

Mit diesen Konstanten und dem jeweils aktuellen Druck Pfill, wird nun zu den Zeitpunkten tn (laufend) der intraabdominale Druck Pabd berechnet. Liegt dieser berechnete Wert um mehr als 2mm Hg über dem Sollwert, dann wird die Haltephase abgebrochen und die Abwärtsrampe wird gestartet. Die Druckvorausberechnung wird in jeder Hauptprogrammschleife durchgeführt, solange sich die Vorrichtung in der Haltephase befindet.

Am Ende der Haltephase von Füllzyklus 2) werden wieder die Größen Pfill und Q abgespeichert und der Vorgang wiederholt sich.

Im folgenden soll ein numerisches Beispiel ohne Beschränkung der Allgemeinheit angegeben werden:

Bei einem Insufflationsbesteck mit einer Verres-Nadel mit einem Innendurchmesser von etwa 3 mm wird erfindungsgemäß typischerweise bis zu einer Gas-Flußrate von 4,5 Liter/min. eine quadratische Abhängigkeit des Druckabfalls von der Flußrate und bei höheren Flußraten eine lineare Abhängigkeit angenommen. Bei einer Gesamt-länge der Schlauchleitung und des Insufflationsbestecks von ca. 2 m beträgt der Druckabfall bei einer Flußrate von 4,5 Liter/min $CO_2$ ca. 16 mm Hg. Diese Werte können natürlich in Abhängigkeit von der Länge des Bestecks und dem Innendurchmesser variieren.

Eine Vorrichtung, die das erfindungsgemäße Prinzip realisiert, kann im Prinzip hinsichtlich des Anschlusses für eine Gasquelle, der Meßeinrichtung mit Sensoren für den Ist-Gasdruck und den Ist-Gasfluß und der Steuereinheit, an der die Ausgangssignale der Sensoren anliegen, und die den einen Druckregler und den Flußregler ansteuert, ähnlich wie bekannte Vorrichtungen aufgebaut sein, wobei lediglich das Ablaufprogramm des Mikroprozessors oder des PC's, der die Funktionen der Vorrichtung steuert, entsprechend dem erfindungsgemäßen Prinzip anzupassen ist. Deshalb wird auf die detaillierte Beschreibung der einzelnen "Hardware-Bauteile" verzichtet.

**Patentansprüche**

1. Vorrichtung zum Insufflieren von Gas in eine Körperhöhle eines menschlichen oder tierischen Körpers, mit

   - einem Anschluß für eine Gasquelle,
   - einer Meßeinrichtung, die Sensoren für den Ist-Gasdruck und den Ist-Gasfluß aufweist, die außerhalb der Körperhöhle angeordnet sind, und
   - einer Steuereinheit, an der die Ausgangssignale der Sensoren anliegen, und die

      - einen Druckregler, der den Druck der Gasquelle auf einen einstellbaren Insufflationsdruck "Soll-Gasdruck" mindert bzw. regelt, und
      - einen Flußregler, der den Gasfluß auf einen einstellbaren Wert "Soll-Gasfluß" regelt, ansteuert, und

   - die Steuereinheit zur Berechnung des Druckabfalls $P_{drop}$ zwischen dem Ort des Drucksensors und der Körperhöhle ausgebildet ist,

   **dadurch gekennzeichnet**, daß-die Steuereinheit zur Berechnung des Druckabfalls $P_{drop}$ zwischen dem Ort des Drucksensors und der Körperhöhle und damit des tatsächlichen Gasdrucks $P_{abd}$ in der Körperhöhle aus dem außerhalb der Körperhöhle gemessenen Gasdruck $P_{fill}$ und dem gemessenen Gasfluß Q stückweise Funktionen verwendet, aufgrund derer der berechnete Druckabfall $P_{drop}$ als Funktion von Q kleiner oder höchstens gleich dem tatsächlichen Druckabfall ist, und

   daß die Steuereinheit Ereignis- und/oder Zeit-gesteuert während der normalen Insufflation laufend den Gasdruck $P_{abd}$ berechnet und aufgrund des berechneten Gasdruckes $P_{abd}$ die Insufflation regelt.

2. Vorrichtung nach Anspruch 1,
   **dadurch gekennzeichnet, daß** die Steuereinheit zur Berechnung von $P_{abd}$ derart ausgestaltet ist, daß

   - bei Flußraten kleiner als eine bestimmte Flußrate $Q_{ums}$ eine quadratische Abhängigkeit des Druckabfalls $P_{drop}$ von der Flußrate Q gilt:

$$P_{abd} = P_{fill} - P_{drop} = P_{fill} - K2*Q^2$$

   - und bei Flußraten größer als die bestimmte Flußrate $Q_{ums}$ eine lineare Abhängigkeit des Druckabfalls $P_{drop}$ von der Flußrate Q gilt:

$$P_{abd} = P_{fill} - P_{drop} = P_{fill} - K1*Q$$

3. Vorrichtung nach Anspruch 2,
**dadurch gekennzeichnet, daß** die Steuereinheit zur Bestimmung der Konstanten K1 und K2 unter Berücksichtigung des Strömungswiderstandes des Insufflationsbesteckes ausgelegt ist.

4. Vorrichtung nach Anspruch 1 oder 2,
**dadurch gekennzeichnet, daß** die Steuereinheit zur Bestimmung der Konstanten K1 und K2 unter Ermittlung der Werte $P_{fill}$ und Q bei im wesentlichen konstantem Gasfluß Q und des Wertes $P_{abd}$ bei einem Gasfluß, der auf einen kleinen Wert oder auf Null abgesenkt ist, ausgelegt ist.

5. Vorrichtung nach Anspruch 4,
**dadurch gekennzeichnet, daß** die Steuereinheit zur Ansteuerung des Flußreglers derart ausgelegt ist, daß der kleine Wert des Gasflußes während der Druckmessung ca. zwischen 10 und 40% des Soll-Gasflusses beträgt.

6. Vorrichtung nach Anspruch 4 oder 5,
**dadurch gekennzeichnet, daß** die Steuereinheit zur Benutzung der Konstanten K1 und K2 für die Berechnung des Drucks $P_{abd}$ in der Körperhöhle lediglich für den nächsten Zyklus, d.h. bis zur nächsten Flußabsenkung, und daß die Steuereinheit zum neuen Ansatz der Konstanten K1 und K2 für jeden Zyklus aus den beim vorherigen Zyklus ermittelten Werten ausgelegt ist.

7. Vorrichtung nach einem der Ansprüche 1 bis 6,
**dadurch gekennzeichnet, daß** die Steuereinheit zur Bestimmung der Flußrate $Q_{ums}$, bei der die Steuereinheit zwischen linearer und quadratischer Abhängigkeit des Druckabfalles $P_{drop}$ von der Flußrate umschaltet, als den Schnittpunkt der linearen und der quadratischen Abhängigkeit des Druckabfalls $P_{drop}$ von der Flußrate Q für die jeweils ermittelten Konstanten K1 und K2 ausgelegt ist.

8. Vorrichtung nach einem der Ansprüche 1 bis 7,
**dadurch gekennzeichnet, daß** die Steuereinheit zur Erzeugung eines Alarms, wenn der Flußsensor den Wert 0 des Gasflusses mißt, ausgelegt ist.

9. Vorrichtung nach einem der Ansprüche 1 bis 8,
**dadurch gekennzeichnet, daß** in an sich bekannter Weise zur Begrenzung des intraabdominalen Druckes ein Entlüftungsventil vorgesehen ist.

10. Vorrichtung nach Anspruch 9,
**dadurch gekennzeichnet, daß** das Entlüftungsventil in dem proximalen Teil eines in die Körperhöhle eingesetzten Instruments vorgesehen ist.

11. Vorrichtung nach Anspruch 9 oder 10,
**dadurch gekennzeichnet, daß** die Steuereinheit zum Schließen des Entlüftungsventils in bestimmten Zeitabständen zur Überprüfung des Körperdruckes ausgeliefert ist.

12. Vorrichtung nach Anspruch 11,
**dadurch gekennzeichnet, daß** die Steuereinheit zur Wahl der Größe der Zeitabstände in Abhängigkeit vom Druck $P_{abd}$ im Körperinneren ausgelegt ist.

13. Vorrichtung nach einem der Ansprüche 9 bis 12,
**dadurch gekennzeichnet, daß** eine Absaugpumpe zur Erhöhung der Absaugleistung vorgesehen ist.

14. Vorrichtung nach Anspruch 13,
**dadurch gekennzeichnet, daß** die Steuereinheit die Leistung der Absaugpumpe in Abhängigkeit vom Druck Pabd im Körperinneren regelt.

15. Vorrichtung nach einem der Ansprüche 9 bis 14,
**dadurch gekennzeichnet, daß** das Entlüftungsventil ein Proportionalventil ist, das die Steuereinheit in Abhängigkeit vom Druck Pabd im Körperinneren regelt.

16. Vorrichtung nach einem der Ansprüche 1 bis 15,
**dadurch gekennzeichnet, daß** in an sich bekannter Weise eine Heizeinrichtung vorgesehen ist, die das zu insufflierende Gas auf eine vorgebbare Temperatur erwärmt.

**17.** Vorrichtung nach Anspruch 16,
**dadurch gekennzeichnet, daß** die Steuereinheit den Gasdruck und den Gasfluß unter Berücksichtigung der vorgegebenen Temperatur des Gases einstellt.

**18.** Vorrichtung nach einem der Ansprüche 1 bis 17,
**dadurch gekennzeichnet, daß** zum Insufflieren des Gases in die Körperhöhle ein endoskopisches Besteck, wie beispielsweise eine Verres-Nadel vorgesehen ist, an der die außerhalb der Körperhöhle angeordneten Sensoren für Gasdruck und Gasfluß vorgesehen sind.

**19.** Vorrichtung nach einem der Ansprüche 1 bis 18,
**dadurch gekennzeichnet, daß** lediglich eine einzige Zuleitung in das Körperinnere führt.

**20.** Vorrichtung nach einem der Ansprüche 1 bis 19,
**dadurch gekennzeichnet, daß** der Druckregler und der Flußregler zu einem Bauteil zusammengefaßt sind.

**Claims**

**1.** Apparatus for insufflating gas into a body cavity of a human or animal body, comprising

- a connection for a gas source;
- a measurement means having sensors for the actual gas pressure and the actual gas flow disposed outside the body cavity; and
- a control unit to which the output signals of the sensors are connected and which controls

  - a pressure regulator for reducing or regulating the pressure of the gas source to a settable insufflation pressure "desired gas pressure"; and
  - a flow regulator for regulating the gas flow to a settable value "actual gas flow";

- the control unit being adapted to calculate the pressure drop $P_{drop}$ between the site of the pressure sensor and the body cavity;

**characterized in that** for calculating the pressure drop $P_{drop}$ between the site of the pressure sensor and the body cavity, and therewith the actual gas pressure $P_{abd}$ in the body cavity, from the gas pressure $P_{fill}$ measured outside the body cavity and the measured gas flow Q, the control unit uses functions selected one at a time, on the basis of which the pressure drop $P_{drop}$ calculated as a function of Q is smaller than or at most equal to the actual pressure drop; and
that during normal insufflation the control unit continually calculates in an event or time-controlled manner the gas pressure $P_{abd}$ and regulates the insufflation on the basis of the calculated gas pressure $P_{abd}$.

**2.** Apparatus according to claim 1,
**characterized in that** for calculating $P_{abd}$ the control unit is adapted in such manner that

- with flow rates smaller than a specified flow rate $Q_{ums}$ a quadratic dependence of the pressure drop $P_{drop}$ on the flow rate Q applies:

$$P_{abd} = P_{fill} - P_{drop} = P_{fill} - K2 \cdot Q^2,$$

- and with flow rates greater than the specified flow rate $Q_{ums}$ a linear dependence of the pressure drop $P_{drop}$ on the flow rate Q applies:

$$P_{abd} = P_{fill} - P_{drop} = P_{fill} - K_1 \cdot Q.$$

**3.** Apparatus according to claim 2,

**characterized in that** the control unit is adapted to determine the constants $K_1$ and $K_2$ while taking into account the flow resistance of the insufflation instrument.

4. Apparatus according to claim 1 or 2,
**characterized in that** the control unit is adapted to determine the constants $K_1$ and $K_2$ by determining the values $P_{fill}$ and Q at a substantially constant gas flow Q and the value $P_{abd}$ at a gas flow which has been reduced to a small value or to zero.

5. Apparatus according to claim 4,
**characterized in that** the control unit is adapted to control the flow regulator in such manner that during the pressure measurement the reduced value of the gas flow is approximately between 10 and 40% of the desired gas flow.

6. Apparatus according to claim 4 or 5,
**characterized in that** the control unit is adapted to use the constants $K_1$ and $K_2$ for calculating the pressure $P_{abd}$ in the body cavity only for the next cycle, i.e. until the next reduction of the flow, and
that the control unit is adapted to set the constants $K_1$ and $K_2$ anew for each cycle from the values determined during the previous cycle.

7. Apparatus according to any one of the claims 1 to 6,
**characterized in that** the control unit is adapted to determine the flow rate $Q_{ums}$ at which the control unit switches between a linear and a quadratic dependence of the pressure drop $P_{drop}$ on the flow rate as being that at the point of intersection of the linear and the quadratic dependence of the pressure drop $P_{drop}$ on the flow rate Q for the constants $K_1$ and $K_2$ as determined in each case.

8. Apparatus according to any one of the claims 1 to 7,
**characterized in that** the control unit is adapted to give an alarm when the flow sensor measures a value 0 for the gas flow.

9. Apparatus according to any one of the claims 1 to 8,
**characterized in that** a relief valve is provided in a manner known per se for limiting the intraabdominal pressure.

10. Apparatus according to claim 9,
**characterized in that** the relief valve is provided in the proximate portion of an instrument inserted into the body cavity.

11. Apparatus according to claim 9 or 10,
**characterized in that** the control unit is adapted to close the relief valve at certain time intervals to check the body pressure.

12. Apparatus according to claim 11,
**characterized in that** the control unit is adapted to select the magnitude of the time intervals in dependence on the pressure $P_{abd}$ inside the body.

13. Apparatus according to any one of the claims 9 to 12,
**characterized in that** a suction pump is provided for increasing the relief performance.

14. Apparatus according to claim 13,
**characterized in that** the control unit regulates the power of the suction pump in dependence on the pressure $P_{abd}$ inside the body.

15. Apparatus according to any one of the claims 9 to 14,
**characterized in that** the relief valve is a proportional valve which is regulated by the control unit in dependence on the pressure $P_{abd}$ inside the body.

16. Apparatus according to any one of the claims 1 to 15,
**characterized in that** a heater is provided in a manner known per se for heating the gas to be insufflated to a specified temperature.

**17.** Apparatus according to claim 16,
**characterized in that** the control unit sets the gas pressure and the gas flow while taking into account the specified temperature of the gas.

**18.** Apparatus according to any one of the claims 1 to 17,
**characterized in that** for insufflating the gas into the body cavity, an endoscopic instrument such as, for example, a Verres needle is provided, on which the sensors for gas pressure and gas flow which are disposed outside the body cavity are provided.

**19.** Apparatus according to any one of the claims 1 to 18,
**characterized in that** only one single line leads into the interior of the body .

**20.** Apparatus according to any one of the claims 1 to 19,
**characterized in that** the pressure regulator and the flow regulator are combined to form one component.

**Revendications**

**1.** Dispositif pour insuffler du gaz dans une cavité du corps d'un être humain ou d'un animal, avec :

- un raccordement à une source de gaz,
- un dispositif de mesure comprenant, agencés à l'extérieur de la cavité corporelle, des capteurs permettant de mesurer la pression de gaz effective et le débit de gaz effectif, et
- une unité de commande recevant les signaux de sortie des capteurs et commandant

  - un régulateur de pression, qui réduit ou régule la pression de la source de gaz à une pression d'insufflation réglable appelée « pression de consigne du gaz », et
  - un régulateur de débit, qui régule le débit de gaz à une valeur réglable appelée « débit de consigne du gaz »,

- l'unité de commande étant conçue pour déterminer la perte de charge $P_{drop}$ entre l'emplacement du capteur de pression et la cavité corporelle,

**caractérisé en ce que**, pour déterminer la perte de charge $P_{drop}$ entre l'emplacement du capteur de pression et la cavité corporelle et, par là même, la pression de gaz effective $P_{abd}$ régnant dans la cavité corporelle à partir de la pression de gaz $P_{fill}$ mesurée à l'extérieur de la cavité corporelle et du débit de gaz Q mesuré, l'unité de commande utilise des fonctions par morceaux telles que la perte de charge calculée $P_{drop}$, qui est fonction de Q, soit inférieure ou égale à la perte de charge effective, et
**en ce que**, pendant l'insufflation normale, l'unité de commande calcule en permanence la pression de gaz $P_{abd}$ de manière commandée par événement ou commandée en temps et régule l'insufflation sur la base de la pression de gaz $P_{abd}$ calculée.

**2.** Dispositif selon la revendication 1, **caractérisé en ce que** l'unité de commande est conçue pour calculer $P_{abd}$ de telle sorte que

- pour les débits inférieurs à un débit donné $Q_{ums}$, il existe une relation quadratique entre la perte de charge $P_{drop}$ et le débit Q :

$$P_{abd} = P_{fill} - P_{drop} = Pfill - K2*Q^2$$

- et pour les débits supérieurs au débit défini $Q_{ums}$, il existe une relation linéaire entre la perte de charge $P_{drop}$ et le débit Q :

$$P_{abd} = P_{fill} - P_{drop} = P_{fill} - K1*Q$$

**3.** Dispositif selon la revendication 2, **caractérisé en ce que** l'unité de commande est conçue pour déterminer les constantes K1 et K2 en tenant compte de la résistance à l'écoulement de l'équipement d'insufflation.

4. Dispositif selon la revendication 1 ou 2, **caractérisé en ce que** l'unité de commande est conçue pour déterminer les constantes K1 et K2 en déterminant les valeurs $P_{fill}$ et Q pour un débit de gaz Q sensiblement constant et la valeur $P_{abd}$ pour un débit de gaz qui est réduit à une valeur faible ou nulle.

5. Dispositif selon la revendication 4, **caractérisé en ce que** l'unité de commande est conçue pour commander le régulateur de débit de telle sorte que la limite inférieure du débit de gaz pendant la mesure de la pression soit comprise environ entre 10 et 40 % du débit de consigne du gaz.

6. Dispositif selon la revendication 4 ou 5, **caractérisé en ce que** l'unité de commande est conçue pour utiliser les constantes K1 et K2 pour le calcul de la pression $P_{abd}$ dans la cavité corporelle uniquement pour le cycle suivant, c'est-à-dire jusqu'à la prochaine baisse de débit, et

   **en ce que** l'unité de commande est conçue pour déterminer de nouvelles constantes K1 et K2 à chaque cycle à partir des valeurs déterminées lors du cycle précédent.

7. Dispositif selon l'une des revendications 1 à 6, **caractérisé en ce que** l'unité de commande est conçue pour déterminer le débit $Q_{ums}$ auquel l'unité de commande bascule entre une relation linéaire et une relation quadratique entre la perte de charge $P_{drop}$ et le débit comme étant le point d'intersection de la relation linéaire et de la relation quadratique entre la perte de charge $P_{drop}$ et le débit Q pour les constantes K1 et K2 chaque fois déterminées.

8. Dispositif selon l'une des revendications 1 à 7, **caractérisé en ce que** l'unité de commande est conçue pour produire un signal d'alarme quand le capteur de débit mesure un débit de gaz nul.

9. Dispositif selon l'une des revendications 1 à 8, **caractérisé en ce qu'**il est prévu d'une manière connue en soi une soupape d'évacuation pour limiter la pression intraabdominale.

10. Dispositif selon la revendication 9, **caractérisé en ce que** la soupape d'évacuation est prévue dans la partie proximale d'un instrument inséré dans la cavité corporelle.

11. Dispositif selon la revendication 9 ou 10, **caractérisé en ce que** l'unité de commande est conçue pour fermer la soupape d'évacuation à des intervalles de temps définis pour contrôler la pression corporelle.

12. Dispositif selon la revendication 11, **caractérisé en ce que** l'unité de commande est conçue pour choisir l'amplitude des intervalles de temps en fonction de la pression $P_{abd}$ régnant à l'intérieur du corps.

13. Dispositif selon l'une des revendications 9 à 12, **caractérisé en ce qu'**une pompe d'aspiration est prévue pour augmenter le débit d'aspiration.

14. Dispositif selon la revendication 13, **caractérisé en ce que** l'unité de commande régule le débit de la pompe d'aspiration en fonction de la pression $P_{abd}$ régnant dans la cavité corporelle.

15. Dispositif selon l'une des revendications 9 à 14, **caractérisé en ce que** la soupape d'évacuation est une soupape proportionnelle commandée par l'unité de commande en fonction de la pression $P_{abd}$ régnant à l'intérieur de la cavité corporelle.

16. Dispositif selon l'une des revendications 1 à 15, **caractérisé en ce qu'**il est prévu, d'une manière connue en soi, un dispositif de chauffage qui chauffe le gaz à insuffler à une température prédéfinissable.

17. Dispositif selon la revendication 16, **caractérisé en ce que** l'unité de commande régule la pression de gaz et le débit de gaz en tenant compte de la température prédéfinie du gaz.

18. Dispositif selon l'une des revendications 1 à 17, **caractérisé en ce que**, pour insuffler le gaz dans la cavité corporelle, il est prévu un équipement endoscopique tel que par exemple une aiguille de Verres sur laquelle sont prévus les capteurs de pression et de débit de gaz agencés à l'extérieur de la cavité corporelle.

19. Dispositif selon l'une des revendications 1 à 18, **caractérisé en ce qu'**un seul conduit mène à l'intérieur de corps.

**20.** Dispositif selon l'une des revendications 1 à 19, **caractérisé en ce que** le régulateur de pression et le régulateur de débit sont réunis en un seul composant.

FIG. 1

EP 0 868 204 B1

FIG. 2